(19) **Europäisches Patentamt European Patent Office Office européen des brevets**

(11) **EP 4 603 509 A1**

(12) **EUROPEAN PATENT APPLICATION**
published in accordance with Art. 153(4) EPC

(43) Date of publication:
20.08.2025 Bulletin 2025/34

(21) Application number: 22961955.6

(22) Date of filing: 23.12.2022

(51) International Patent Classification (IPC):
C07K 16/28 (2006.01)    C12N 15/13 (2006.01)
A61K 39/395 (2006.01)    A61P 35/00 (2006.01)

(86) International application number:
PCT/CN2022/141447

(87) International publication number:
WO 2024/077777 (18.04.2024 Gazette 2024/16)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 13.10.2022 CN 202211255482

(71) Applicant: Shenzhen Baishitong Technology Development Company Limited
Shenzhen, Guangdong 518051 (CN)

(72) Inventors:
• LOU, Jing
Shenzhen, Guangdong 518051 (CN)
• CHEN, Jianhe
Shenzhen, Guangdong 518051 (CN)
• SU, Dongmei
Shenzhen, Guangdong 518051 (CN)
• JIN, Zheng
Shenzhen, Guangdong 518051 (CN)
• LV, Yunying
Shenzhen, Guangdong 518051 (CN)

• ZHANG, Ruolan
Shenzhen, Guangdong 518051 (CN)
• PEI, Ruochen
Shenzhen, Guangdong 518051 (CN)
• OU, Yanmei
Shenzhen, Guangdong 518051 (CN)
• QU, Xiao
Shenzhen, Guangdong 518051 (CN)
• XIE, Xie
Shenzhen, Guangdong 518051 (CN)
• ZHANG, Jing
Shenzhen, Guangdong 518051 (CN)
• ZENG, Lin
Shenzhen, Guangdong 518051 (CN)

(74) Representative: Kailuweit & Uhlemann
Patentanwälte
Partnerschaft mbB
Bamberger Straße 49
01187 Dresden (DE)

Remarks:
The complete document including Reference Table(s) and the Sequence Listing(s) can be downloaded from the EPO website

(54) **MULTIFUNCTIONAL RECOMBINANT ANTIBODY, AND PREPARATION METHOD AND USE THEREFOR**

(57) Disclosed in the present disclosure are a multifunctional recombinant antibody, a preparation method, and use thereof. Provided are a monoclonal antibody that specifically recognizes human CD276 protein, a humanized recombinant antibody thereof, and further a multifunctional recombinant antibody that recognizes human CD276 and has a function of IL 15, which can effectively enhance the killing effect on tumor cells. Meanwhile, by introducing a mutated human IgG1 constant region, a modified CD276-targeting monoclonal antibody-IL15 bifunctional molecule is obtained, such that the toxicity of the antibody can be effectively reduced and the safety is improved. The antibody in the present disclosure can recognize CD276 protein on various tumor cells, thereby having a killing effect on various tumor cells. Moreover, the reconstructed CD276-targeting IL15 recombinant antibody has the advantages of short drug metabolic cycle, low toxicity, and high safety, and thus has good application prospects.

EP 4 603 509 A1

Binding **hCD276**-ECD (**147#**)

| | 147# |
|---|---|
| EC50 | 38.35 |

FIG. 1

**Description**

**FIELD OF TECHNOLOGY**

The present disclosure relates to the technical field of biotechnology, and relates to an Fc-mutated CD276-targeting IL 15 multifunctional recombinant antibody, a preparation method and use thereof, in particular to a multifunctional recombinant antibody that recognizes CD276 protein, has an IL15 function, and exhibits reduced *in vivo* toxicity as well as use thereof.

**BACKGROUND**

CD276, also known as B7-H3, is a type I transmembrane protein, which is a member of the B7 immune co-stimulatory and co-inhibitory family and has immunoregulatory functions. As an immunoregulatory related receptor protein, its ligands have not yet been conclusively identified. In normal human tissues, CD276 is expressed at low levels, and typically expressed on activated macrophages and monocytes. CD276 plays an inhibitory role on T cells, preventing excessive immune activation.

CD276 is widely expressed in various malignant tumors, including lung cancer, liver cancer, colorectal cancer, gastric cancer, breast cancer, pancreatic cancer, and renal cancer. The expression of CD276 on tumor cells promotes tumor progression and leads to poor prognosis, as CD276 can suppress anti-tumor immunity in the tumor microenvironment.

Therefore, CD276 can serve as a reliable target for tumor treatment.

Firstly, CD276 as an immune checkpoint molecule, blocking its function can enhance the body's anti-tumor immunity.

Secondly, CD276 is widely expressed on tumor cells. Specific antibodies targeting CD276 can kill tumor cells through mechanisms such as ADCC (antibody-dependent cellular cytotoxicity), CDC (complement-dependent cytotoxicity), and ADCP (antibody-dependent cellular phagocytosis).

Thirdly, the specific expression of CD276 on tumor cells allows for targeting killing of tumor cells through ADC (antibody-drug conjugate).

IL15 is a cytokine expressed by various cells, including monocytes, macrophages, epithelial cells, and fibroblasts and excluding T lymphocytes. Unlike many other cytokines, IL15 typically exerts its effects without being secreted from cells. Specifically, IL15 binds with IL15R$\alpha$ and is localized to the specific cell membrane , thereby stimulating nearby effector cells, mainly NK (natural killer) cells and CD8+ T cells. IL15 is closely related to IL2. Upon forming a complex with IL15R$\alpha$, IL15 can further bind to the $\beta/\gamma$ receptor shared with IL2, mediating biological activity. In terms of anti-tumor activity, a key advantage of IL15 over IL2 is that EL15/R$\alpha$ does not stimulate the proliferation of Treg cells.

Currently, several IL15-related molecules are in development for the treatment of malignant tumors. The most advanced of these is ALT-803, which is a complex formed by IL15 and IL15R$\alpha$ sushi-hFc1. Multiple clinical studies have shown that ALT-803 is effective against various tumors, including melanoma. However, it also causes severe toxic side effects, primarily including liver dysfunction, hypotension, and fever.

**SUMMARY**

The present disclosure provides a monoclonal antibody that specifically recognizes CD276 protein; a hybridoma cell that secretes the monoclonal antibody; a recombinant antibody obtained by humanizing the monoclonal antibody; and a multifunctional recombinant antibody that recognizes human CD276 protein and has a human IL15 function, which can be used for the treatment of malignant tumors. Meanwhile, a modified specific monoclonal antibody-IL15 bifunctional molecule is provided, which effectively reduces the toxicity of the antibody while maintaining the anti-tumor activity.

The technical solution of the present disclosure is as follows:

The present disclosure provides a monoclonal antibody, the monoclonal antibody recognizes human CD276-ECD protein; the monoclonal antibody comprises a heavy chain variable region having an amino acid sequence of SEQ ID NO: 5, and a light chain variable region having an amino acid sequence of SEQ ID NO: 7.

The present disclosure prepares a murine monoclonal antibody, named 147#, which specifically recognizes human CD276 protein by immunizing mice with recombinant human CD276-ECD protein. The amino acid sequences of the heavy chain variable region and the light chain variable region of the murine monoclonal antibody are obtained through biological analysis. The antibody can effectively bind to CD276 protein. Through biological analysis, the heavy chain variable region of the monoclonal antibody corresponds to 122 amino acid residues, and the light chain variable region of the monoclonal antibody corresponds to 107 amino acid residues.

Furthermore, the present disclosure also provides a recombinant antibody, the recombinant antibody is obtained by humanizing the monoclonal antibody 147 #, the recombinant antibody comprises a heavy chain variable region having an amino acid sequence of SEQ ID NO: 21, and a light chain variable region having an amino acid sequence of SEQ ID NO: 22.

As a preferred embodiment of the present disclosure, the amino acid sequence of the heavy chain of the recombinant antibody is as shown in SEQ ID NO: 23, and the amino acid sequence of the light chain of the recombinant antibody is as shown in SEQ ID NO: 24.

The inventor of the present disclosure further analyzed the sequence of the obtained murine monoclonal antibody 147# and replaced the CDR regions of a humanized template with those of 147#. The heavy chain variable region (VH) was then recombined with the human IgG1 constant region, and the light chain variable region (VL) was recombined with the human kappa chain constant region. Based on the three-dimensional structure of this antibody, buried residues, residues that directly interact with the CDR regions, and residues that significantly affect the conformations of the VL and VH of antibodies were subjected to revertant mutations. This ultimately resulted in the generation of a humanized recombinant antibody, named hu147#. The amino acid sequences of the heavy chain variable region, light chain variable region, heavy chain, and light chain of the humanized recombinant antibody hu147# are as described above.

Furthermore, the present disclosure provides a multifunctional recombinant antibody, the multifunctional recombinant antibody comprises a heavy chain, the heavy chain of the multifunctional recombinant antibody comprises a human CD276-targeting antibody functional region, a human IgG1 constant functional domain, a IL15 functional region, and several non-functional amino acid fragments for linking the functional domain and the functional regions; the human CD276-targeting antibody functional region comprises the amino acid sequence of the heavy chain variable region of the recombinant antibody described above.

The IL15 functional region recognizes the functional domain of the human IL2/IL15β/γ receptor.

The inventors of the present disclosure further modified the obtained humanized recombinant antibody by linking the sequence of the heavy chain of the antibody to a sequence having an IL 15 function, obtaining the multifunctional recombinant antibody that recognizes human CD276 protein and has an IL15 function.

As a preferred embodiment of the present disclosure, the human IgG1 constant functional domain is the human IgG1 constant region having an amino acid sequence of SEQ ID NO: 8.

More preferably, the human IgG1 constant functional domain is a mutated human IgG1 constant region, and the mutated human IgG1 constant region has an amino acid sequence of SEQ ID NO: 25.

The inventors of the present disclosure have developed a CD276-targeting IL15 recombinant antibody with a shorter metabolic cycle, reduced toxicity, and improved safety by using a mutated human IgG1 constant region sequence.

As a preferred embodiment of the present disclosure, the amino acid sequence of the human IL15 functional region is as shown in SEQ ID NO: 28.

More preferably, the amino acid sequence of the human IL15 functional region is as shown in SEQ ID NO: 29.

The amino acid sequence of the preferred human IL15 functional region, as shown in SEQ ID NO: 29, is the single-chain IL15 (i.e., IL15sc) formed by linking human IL15Rsushi with human IL15 via a (GGGGS)$_6$ linker.

As a preferred embodiment of the present disclosure, the non-functional amino acid fragments for linking the functional domain and regions in the CD276-targeting IL15 recombinant antibody is a GGGGS repeat. This linker (GGGGS repeat) plays a crucial role in constructing stable and biologically active fusion proteins, ensuring correct protein folding, maintaining biological activity, and increasing protein yield.

More preferably, the GGGGS repeat is (GGGGS)$_3$.

As a preferred embodiment of the present disclosure, the amino acid sequence of the human IgG1 constant functional domain of the multifunctional recombinant antibody is as shown in SEQ ID NO: 8; the multifunctional recombinant antibody comprises a heavy chain having an amino acid sequence of SEQ ID NO: 30 and a light chain having an amino acid sequence of SEQ ID NO: 24.

The present disclosure prepares a multifunctional antibody that recognizes CD276 protein and has an IL15 function, named SPGL007.

More preferably, the human IgG1 constant functional domain is a mutated human IgG1 constant region, and the mutated human IgG1 constant region has an amino acid sequence of SEQ ID NO: 25; the heavy chain of the multifunctional recombinant antibody has an amino acid sequence of SEQ ID NO: 31; and the light chain of the multifunctional recombinant antibody has an amino acid sequence of SEQ ID NO: 24.

The multifunctional recombinant antibody obtained from the preferred sequence of the multifunctional recombinant antibody comprises a mutated human IgG1 constant region sequence, named SPGL008. The multifunctional recombinant antibody SPGL008 not only effectively recognizes CD276 in various tumor cells but also effectively inhibits the growth of multiple cancer cells, with a shorter half-life, lower toxicity, and improved safety.

Furthermore, the present disclosure also provides a nucleotide sequence encoding the monoclonal antibody, the recombinant antibody, or the multifunctional recombinant antibody.

Based on the amino acid sequence of the monoclonal antibody, the recombinant antibody, or the multifunctional recombinant antibody, the corresponding nucleotide sequence encoding the monoclonal antibody, the recombinant antibody, or the multifunctional recombinant antibody can be obtained.

As a preferred embodiment of the present disclosure, the nucleotide sequence encoding the heavy chain variable region of the monoclonal antibody is as shown in SEQ ID NO: 4, and the nucleotide sequence encoding the light chain variable region of the monoclonal antibody is as shown in SEQ ID NO: 6.

Furthermore, the present disclosure also provides an expression vector comprising the nucleotide sequence.

Using molecular biology methods, the expression vector comprising the nucleotide sequence can be constructed.

Furthermore, the present disclosure also provides a host cell comprising the expression vector.

Using cell biology methods, the host cell that effectively expresses the antibody protein can be constructed using the expression vector.

Furthermore, the present disclosure also provides use of the monoclonal antibody, the recombinant antibody, the multifunctional recombinant antibody, the nucleotide sequence, the expression vector, or the host cell in the preparation of a biopharmaceutical composition for the treatment of tumors.

Furthermore, the present disclosure also provides a biopharmaceutical composition comprising one or more of the monoclonal antibody, the recombinant antibody, the multifunctional recombinant antibody, the nucleotide sequence, the expression vector, and the host cell.

Furthermore, the present disclosure provides a method for preparing the monoclonal antibody, the recombinant antibody, or the multifunctional recombinant antibody, including the following steps:

(1) obtaining an expression vector comprising a gene fragment of the monoclonal antibody, the recombinant antibody, or the multifunctional recombinant antibody (CD276-targeting IL15 recombinant antibody) by artificial synthesis or molecular biology methods;

(2) transfecting the expression vector into cells for expression of protein;

(3) purifying the protein to obtain the monoclonal antibody, the recombinant antibody, or the multifunctional recombinant antibody (CD276-targeting IL15 recombinant antibody).

By constructing the expression vector comprising the nucleotide sequence encoding the heavy chain or the light chain of the antibody, transfecting suitable cells for expression and performing purification of protein, the corresponding antibody can be obtained.

As a preferred embodiment of the present disclosure, in step (1), the obtaining the expression vector comprising the gene fragment of the monoclonal antibody, the recombinant antibody, or the multifunctional recombinant antibody by artificial synthesis or molecular biology methods includes inserting the nucleotide sequence encoding the heavy chain or the light chain of the antibody into the pcDNA3.4 expression vector.

As a preferred embodiment of the present disclosure, in step (2), the cells used for expression of the protein are Expi-293F cells.

As a preferred embodiment of the present disclosure, in step (3), the purifying the protein is using Protein G affinity chromatography.

The present disclosure has the following technical effects:

The present disclosure provides a monoclonal antibody that specifically recognizes human CD276 protein, as well as a humanized recombinant antibody thereof. Based on the above, the inventors further develops a multifunctional recombinant antibody that not only recognizes human CD276 but also has an IL15 function by utilizing the CD276-specific recombinant antibody. Compared to the monoclonal antibody, the multifunctional recombinant antibody can effectively enhance the killing effect on tumor cells. Additionally, by introducing a mutated human IgG1 constant region, a modified specific CD276 monoclonal antibody-IL15 bifunctional molecule is obtained. This bifunctional molecule not only retains *in vivo* anti-tumor activity but also significantly shortens the metabolic cycle, such that the toxicity of the antibody can be effectively reduced and the safety is improved. The multifunctional recombinant antibody of the present disclosure can recognize CD276 protein on various tumor cells, demonstrating great application prospects.

## BRIEF DESCRIPTION OF DRAWINGS

FIG. 1 shows the results of binding activity of murine antibody 147 # to human CD276-ECD protein, which are obtained by the ELISA method.

FIG. 2 shows the results of binding activity of murine monoclonal antibody to CD276, which are obtained by flow cytometry.

FIG. 3 shows the results of binding activity of 147 # to human CD276-ECD, which are obtained by Western Blot method.

FIGs. 4 and 5 show the binding results of 147 # to peptides within the Ig-like C2-type1 region of human CD276 domain, which are obtained by the ELISA method.

FIG. 6 shows the results of binding activity of monoclonal antibody 147 # to crab-eating macaque CD276-ECD antigen.

FIG. 7 shows the results of binding activity of humanized antibody hu147 and mutated humanized antibody hu147mu to human CD276-ECD, which are obtained by the ELISA method.

FIG. 8 shows the results of binding activity of SPGL008 and SPGL007 to human CD276-ECD, which are obtained by the ELISA method.

FIG. 9 shows the binding results of SPGL008 to both human CD276 and human CD122/132.

FIGs. 10A and 10B show the results of the cell proliferation-promoting activity of SPGL008.

FIG. 11 shows the results of SPGL008 inhibiting the growth of mouse colorectal cancer cell MC38 xenograft tumors.

FIG. 12 shows the results of SPGL008 inhibiting the growth of human lung cancer cell NCI H1975 xenograft tumors.

FIG. 13 shows the results of SPGL008 combined with HER2 monoclonal antibody inhibiting the growth of human breast cancer cell JIMT-1 xenograft tumors in nude mice.

FIG. 14 shows the metabolism results of SPGL008 in HuFcRn transgenic mice.

FIG. 15 shows the binding results of SPGL008 to various tumor cells, which are obtained by flow cytometry.

FIG. 16 shows the results of 0 week-storage of SPGL008.

FIG. 17 shows the results of 5 weeks-storage of SPGL008 at 4°C.

FIG. 18 shows the results of 5 weeks-storage of SPGL008 at 37°C.

## DETAILED DESCRIPTION

In order to better illustrate the objectives, technical solutions, and advantages of the present disclosure, the present disclosure will be further described below with reference to the accompanying drawings and specific embodiments.

**Embodiment 1 Immunization of Animals with Antigen, and Preparation and Screening of Hybridomas**

Step 1: Immunization of Mice with Antigen

Recombinant human CD276-ECD protein (purchased from Acro BIOSYSTEMS, amino acid sequence as shown in SEQ ID NO: 1) was administered subcutaneously to perform immunization on Balb/c mice. On Day 1, human CD276-ECD protein was emulsified and mixed thoroughly with complete Freund's adjuvant (CFA, Sigma) and subcutaneously injected into Balb/c mice (50 μg/mouse). On Day 14 and Day 36, human CD276-ECD protein was emulsified and mixed fully with incomplete Freund's adjuvant (IFA, Sigma) and subcutaneously injected into Balb/c mice to booster immunization (50 μg/mouse). On Day 50, 50 μg of human CD276-ECD protein was injected intraperitoneally into the mice to induce a strong immune response. Three to four days later, the spleens of the mice were taken for fusion experiments.

Step 2: Preparation and Screening of Hybridomas

Three to four days after the last immunization of the mice, the conventional hybridoma techniques were used to fuse mouse spleen cells with mouse myeloma cells SP2/0 via PEG (PEG1450, purchased from Sigma). The fused cells were suspended evenly in complete culture medium, which was prepared by mixing RPMI 1640 and DMEM F12 medium at a ratio of 1:1, and adding 1% Glutamine (purchased from Gibco), 1% Sodium Pyruvate (purchased from Gibco), 1% MEM-NEAA (Minimal Essential Medium-Non-Essential Amino Acid Solution, purchased from Gibco), 1% Penicillin-Streptomycin (purchased from Gibco), 50 $\mu$M $\beta$-mercaptoethanol (purchased from Gibco), and 20% FBS (purchased from Gibco). The suspension was seeded at $10^5$ cells/100 $\mu$L/well in a 96-well culture plate and then cultured overnight. The next day, 100 $\mu$L of complete medium containing 2×HAT (purchased from Sigma) was added to each well, making the total volume in each well 200 $\mu$L (containing 1×HAT). After 7 to 12 days, the supernatant was collected, and the hybridomas with positive binding activity to human CD276-ECD protein were selected using an indirect enzyme-linked immunosorbent assay (ELISA).

Specifically, the indirect enzyme-linked immunosorbent assay (ELISA) method for screening the hybridomas with positive binding activity to human CD276-ECD protein was as follows: Recombinant human CD276-ECD protein was diluted to 1 $\mu$g/mL using the coating solution (50 mM carbonate coating buffer, pH 9.6), and 100 $\mu$L was added to each well of the ELISA plates. The plates were coated with the recombinant human CD276-ECD protein and incubated at 4°C overnight. Afterward, the plates were washed 3 times with PBST, and 200 $\mu$L of blocking solution (2% BSA-PBST) was added to each well and incubated at 37°C for 1 hour. Then, the plates were washed once with PBST for use. The collected hybridoma supernatant was added to the blocked plates at 100 $\mu$L per well and incubated at 37°C for 1 hour, followed by 3 additional washes with PBST. HRP-conjugated goat anti-mouse IgG secondary antibody (purchased from Abcam, catalog number ab6789) was added, and the plates were incubated at 37°C for 30 minutes. After 5 more washes with PBST, residual droplets were removed by blotting on absorbent paper. Then, 100 $\mu$L of TMB substrate (purchased from KPL) was added to each well, and the plates were incubated at room temperature (20 $\pm$ 5°C) in the dark for 5 minutes. Finally, 50 $\mu$L of 2 M $H_2SO_4$ was added to each well to terminate the substrate reaction, and the OD value at 450 nm was read using a microplate reader to analyze the binding ability of the to-be-tested antibody to the target antigen, i.e., human CD276-ECD protein.

The 10 hybridoma cell lines obtained by screening were expanded in serumcontaining complete culture medium. The hybridoma cells were centrifuged, and added to serum-free medium (SFM), adjusting the cell density to 1-2 $\times 10^7$ cells/mL. The cells were cultured at 37°C for two weeks under 5% $CO_2$. After centrifugation, the supernatant was collected, and the antibody was purified using Protein G affinity chromatography. A murine monoclonal antibody against human CD276-ECD protein was obtained and named 147#.

**Embodiment 2 Determination of the Binding Activity of Murine Antibody 147# to Human CD276-ECD Protein by ELISA Method**

The binding ability of the murine antibody to human CD276-ECD protein was determined using an indirect enzyme-linked immunosorbent assay (ELISA). The specific method was as follows: The plates were pre-coated with human CD276-ECD protein diluted to 2 $\mu$g/mL in the coating solution (50 mM carbonate coating buffer, pH 9.6) and incubated at 4°C overnight. The plates were then blocked with 5% skim milk powder at 37°C for 2 hours. Afterward, the plates were washed 3 times with PBST, and the to-be-tested antibody, diluted using 1% BSA-PBST at various gradients, was added to the blocked plates at 100 $\mu$L per well. The plates were incubated at 37°C for 1 hour. After washing the plates 3 times with PBST, HRP-conjugated goat anti-mouse IgG secondary antibody (purchased from Millipore) was added, and the plates were incubated at 37°C for 30 minutes. After 3 more washes with PBST, residual droplets were removed by blotting on absorbent paper. Then, 100 $\mu$L of TMB substrate (purchased from KPL) was added to each well, and the plates were incubated at room temperature (20 $\pm$ 5°C) in the dark for 5 minutes. Finally, 50 $\mu$L of 2 M $H_2SO_4$ was added to each well to terminate the substrate reaction, and the OD value at 450 nm was read using a microplate reader to analyze the binding ability of the to-be-tested antibody to the target antigen, i.e., human CD276-ECD protein.

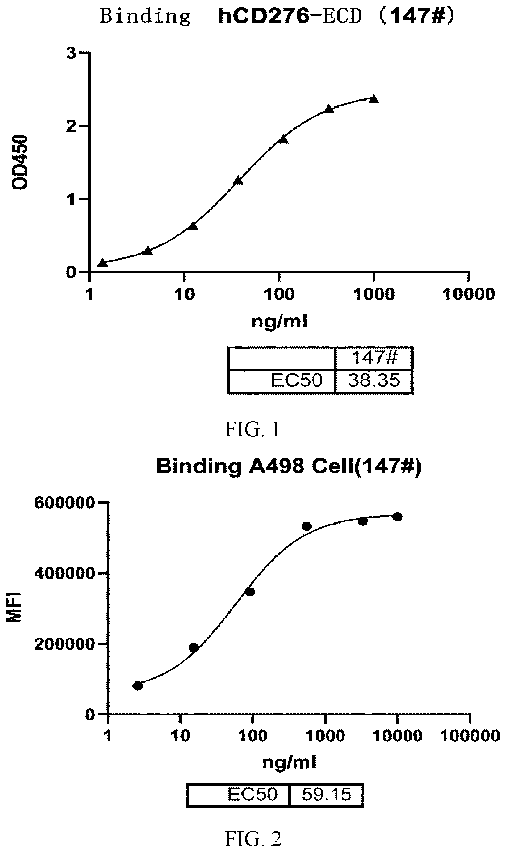

The results, shown in FIG. 1, demonstrated that antibody 147# exhibited good binding activity to human CD276-ECD protein, with an $EC_{50}$ of 38.35 ng/mL, i.e., 0.26 nM.

**Embodiment 3 Determination of the Binding Activity of Murine Monoclonal Antibody to CD276 by Flow Cytometry Method**

The binding affinity of antibody 147# to human renal cancer cells A498 was measured using fluorescence activated cell sorting (FACS) technology.

In this experiment, human renal cancer cells A498 were used as the target cells. A 100 $\mu$L of antibody 147#, serially diluted in six-fold gradients (four consecutive dilutions starting from 10,000 ng/mL), was used as the primary antibody. Each dilution of antibody 147# (with a highest working concentration of 5 $\mu$g/mL and a lowest working concentration of 23 ng/mL) was incubated with 1×$10^5$ A498 cells suspended in 100 $\mu$L of RPMI-1640 serum-free medium (purchased from Gibco, catalog number 22400089) at 4°C for 1 hour. The cells were washed twice with PBS to remove those 147# unbinding to the cells, and then incubated with 100 $\mu$L of 2 $\mu$g/mL Alexa Fluor 488-labeled anti-mouse fluorescent secondary antibody (purchased from Thermo, Invitrogen, catalog number A11001) at 4°C for 30 minutes. The cells were washed twice with PBS to remove those secondary antibody unbinding to the cells and then resuspended in 100 µL PBS. The binding affinity of 147# to these cells was determined using a flow cytometer, and the data were analyzed and fitted using GraphPad Prism 6 software.

The results, shown in FIG. 2, indicated that 147# can specifically bind to human renal cancer cells A498, which highly express CD276 on their surface, with an $EC_{50}$ of 59.15 ng/mL, i.e., 0.39 nM.

**Embodiment 4 Determination of Epitope of 147# on Antigen**

**4.1 Determination of the Binding Ability of 147# to Reduced and Denatured CD276-ECD**

The binding ability of 147# to reduced and denatured human CD276-ECD was measured using Western Blot.

Reduced and denatured human CD276-ECD was subjected to SDS-PAGE electrophoresis (400 ng per lane) and then transferred to a PVDF membrane by electroblotting. The membrane was blocked with 1% BSA-TBST at room temperature for 1 hour with shaking. Afterward, 1 µg/mL of the murine antibody 147# (diluted in 1% BSA-PBST) was added, followed by incubation at room temperature for 2 hours with shaking. The membrane was washed three times with PBST. Then, HRP-conjugated goat anti-mouse IgG secondary antibody (purchased from Abcam, catalog number ab6789, diluted 10,000 folds in 1% BSA-PBST according to the manufacturer's instructions) was added, and the membrane was incubated for 1 hour at room temperature with shaking. After washing three times with PBST, an appropriate amount of Pierce™ ECL Western blot substrate solution (purchased from Thermo, catalog number 32209) was added to the PVDF membrane. The membrane was incubated at room temperature in the dark and then automatical imaging was performed using a biomolecular imager (purchased from Thermo, model CL1500).

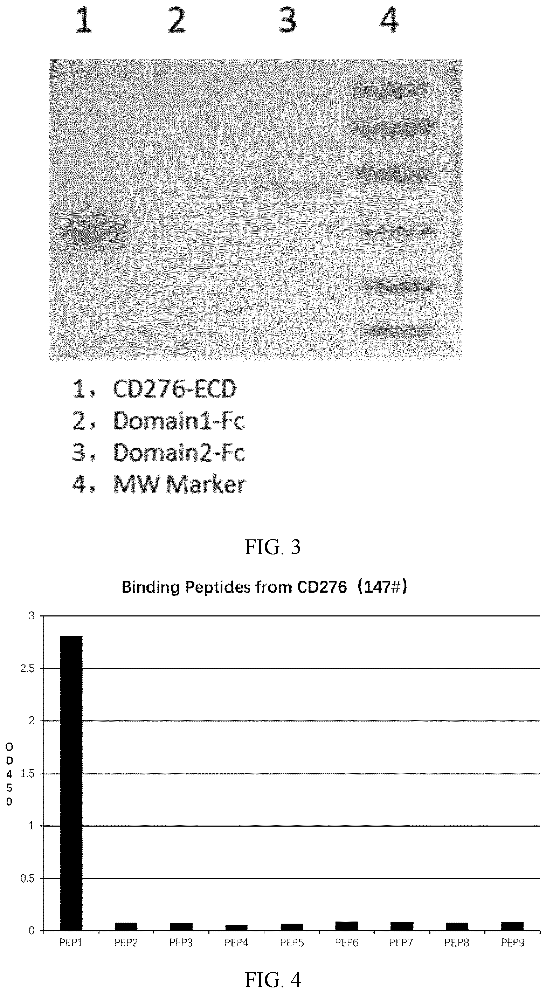

The results were shown in FIG. 3. A specific immunoblot was observed at the CD276-ECD target position, indicating that 147# can specifically bind to reduced and denatured human CD276-ECD. This suggests that the epitope recognized by antibody 147# on human CD276-ECD protein is a linear epitope.

**4.2. Determination of the Binding Region of Monoclonal Antibody 147# to the Target Antigen**

The binding region of 147# to human CD276-ECD was determined using Western blot and ELISA.

To confirm the binding epitope of 147# on CD276, the gene for human CD276 extracellular domain (CD276-ECD) was obtained from the literature and NCBI. The CD276-ECD includes the functional domains Ig-like V-type 1 (Domain 1, amino acid sequence as shown in SEQ ID NO: 16) and Ig-like C2-type 1 (Domain 2, amino acid sequence as shown in SEQ ID NO: 17). The functional domains Ig-like V-type 1 and Ig-like C2-type 1 were then linked to the human Fc region (amino acid sequence as shown in SEQ ID NO: 18) to form the V-type 1-Fc (amino acid sequence as shown in SEQ ID NO: 19) and C2-type 1-Fc (amino acid sequence as shown in SEQ ID NO: 20), respectively. The V-type 1-Fc and C2-type 1-Fc were constructed into the pcDNA3.4 expression vector, transfected into Expi-293F cells for expression of proteins, and the proteins were purified using Protein A to obtain Fc fusion proteins. Later, the binding of 147# to the functional domains Ig-like V-type 1 and Ig-like C2-type 1 of human CD276-ECD was assessed by Western blot following the method described in Section 4.1.

The Western blot results, shown in FIG. 3, indicated that 147# can bind specifically to the second functional domain of human CD276-ECD, i.e., the Ig-like C2-type 1.

**4.3. Confirmation of the Peptide Bound by Monoclonal Antibody 147# on the Target Antigen**

The binding ability of monoclonal antibody 147# to peptides in the Ig-like C2-type 1 domain of human CD276 was detected using ELISA, and the epitope recognized by 147# on human Ig-like C2-type 1 was further confirmed.

Based on the results from sections 4.1 and 4.2, it has been confirmed that 147# can bind to denatured CD276-ECD and the Ig-like C2-type 1 domain. The epitope recognized by 147# was determined to be a linear epitope. The precise location of the epitope bound by antibody 147# can be further clarified by using synthesized peptides via ELISA.

The human Ig-like C2-type 1 domain was divided into nine segments which have overlapped portions, and each segment was synthesized to an N-terminal biotin-labeled peptide, specifically:

1.bio-PSMTLEPNKD LRPGDTVTI (145-164);

2.bio-LRPGDTVTI TCSSYQGYPE (155-174);

3.bio-TCSSYQGYPE AEVFWQDGQG (165-184);

4.bio-AEVFWQDGQG VPLTGNVTTS (175-194);

5.bio-VPLTGNVTTS QMANEQGLFD (185-204);

6.bio-QMANEQGLFD VHSILRVVLG (195-214);

7.bio-VHSILRVVLG ANGTYSCLVR (205-224);

8.bio-ANGTYSCLVR NPVLQQDAHS (215-234);

9.bio-NPVLQQDAHS SVTIT (225-240).

The binding activity of 147# to the above 9 peptides was measured by ELISA, and the results were shown in FIG. 4. The results indicated that 147# specifically bound to peptide 1, which corresponds to the peptide composed of amino acids 145-154 at the N-terminus of human CD276-ECD, illustrating that the epitope of 147# is located between P145 and I164 of human CD276 protein.

Furthermore, by performing alanine scanning (where non-alanine amino acid sites are individually mutated to alanine), 20 N-terminal biotin-labeled peptides were synthesized (the mutated amino acid sites are underlined), as follows:

1-1.bio- ASMTLEPNKD LRPGDTVTI (145-164);

1-2.bio-P AMTLEPNKD LRPGDTVTI (145-164);

1-3.bio-PS ATLEPNKD LRPGDTVTI (145-164);

1-4.bio-PSM ALEPNKD LRPGDTVTI (145-164);

1-5.bio-PSMT AEPNKD LRPGDTVTI (145-164);

1-6.bio-PSMTL APNKD LRPGDTVTI (145-164);

1-7.bio-PSMTLE ANKD LRPGDTVTI (145-164);

1-8.bio-PSMTLEP AKD LRPGDTVTI (145-164);

1-9.bio-PSMTLEPN AD LRPGDTVTI (145-164);

1-10.bio-PSMTLEPNK ALRPGDTVTI (145-164);

1-11.bio-PSMTLEPNKD ARPGDTVTI (145-164);

1-12.bio-PSMTLEPNKD L APGDTVTI (145-164);

1-13.bio-PSMTLEPNKD LR AGDTVTI (145-164);

1-14.bio-PSMTLEPNKD LRP ADTVTI (145-164);

1-15.bio-PSMTLEPNKD LRPG ATVTI (145-164);

1-16.bio-PSMTLEPNKD LRPGD AVTI (145-164);

1-17.bio-PSMTLEPNKD LRPGDT ATI (145-164);

1-18.bio-PSMTLEPNKD LRPGDTV AI (145-164);

1-19.bio-PSMTLEPNKD LRPGDTVT A (145-164);

1-20.bio-PSMTLEPNKD LRPGDTVTI (145-164).

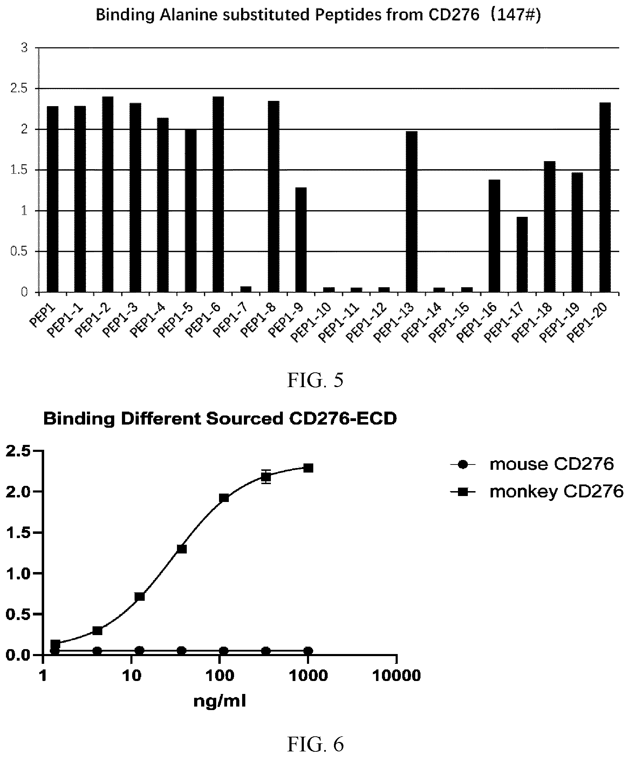

The results shown in FIG. 5 indicated that 147# exhibited negligible binding to peptides 1-7, 1-10, 1-11, 1-12, 1-14, and 1-15, and showed reduced binding to peptides 1-9 and 1-17. For other peptides, the binding was almost similar to the original peptide (PEP1). These results suggest that the amino acids P/D/L/R/G/D in peptide PEP1 are the most critical for 147# binding, followed by K/V. Therefore, the critical binding sites bound by monoclonal antibody 147# are P151/D154/L155/R156/G157/D158 in human CD276, with K153/V161 being the sub-critical binding sites.

**Embodiment 5 Determination of Binding Activity of Monoclonal Antibody 147# to Crab-eating Macaque CD276-ECD Antigen**

An indirect enzyme-linked immunosorbent assay (ELISA) was used to determine the binding ability of antibody147# to crab-eating macaque CD276-ECD (purchased from Acro BIOSYSTEMS, amino acid sequence as shown in SEQ ID NO: 2) and mouse CD276-ECD (purchased from Acro BIOSYSTEMS, amino acid sequence as shown in SEQ ID NO: 3). The specific method was as follows: The plates were pre-coated with crab-eating macaque and mouse CD276-ECD proteins diluted to 2 $\mu$g/mL in the coating solution (50 mM carbonate coating buffer, pH 9.6) and incubated at 4°C overnight. The plates were then blocked with 5% skim milk powder at 37°C for 2 hours. Afterward, the plates were washed 3 times with PBST, and the to-be-tested antibody, diluted in 1% BSA-PBST at various gradients, was added to the blocked plates at 100 $\mu$L per well. The plates were incubated at 37°C for 1 hour. After washing the plates 3 times with PBST, HRP-conjugated goat anti-mouse IgG secondary antibody (purchased from Millipore) was added, and the plates were incubated at 37°C for 30 minutes. After 3 more washes with PBST, residual droplets were removed by blotting on absorbent paper. Then,100 $\mu$L of TMB substrate (purchased from KPL) was added to each well, and the plates were incubated at room temperature (20 $\pm$ 5°C) in the dark for 5 minutes. Finally, 50 $\mu$L of 2 M $H_2SO_4$ was added to each well to terminate the substrate reaction, and the OD value at 450 nm was read using a microplate reader to analyze the binding ability of the to-be-tested antibody to the target antigens, i.e., crab-eating macaque and mouse CD276-ECD proteins.
The results, as shown in FIG. 6, indicated that the $EC_{50}$ of antibody 147# for binding to crab-eating macaque CD276-ECD protein was 30.16 ng/mL, i.e., 0.2 nM, which is nearly consistent with EC50 for binding to human CD276-ECD protein (38.35 ng/mL). However, antibody 147# did not bind to mouse CD276-ECD protein.

**Embodiment 6 Preparation of Humanized Antibody hu147**

In this embodiment, the heavy chain variable region and light chain variable region of hybridoma 147# were obtained through molecular biology methods, and a chimeric antibody was further constructed.
The RNA of hybridoma 147# was extracted by Trizol, and reverse transcribed to obtain cDNA. Then, the cDNA was used as a template, and PCR was performed with degenerate primers for the heavy chain and light chain of the murine antibody (sequences of the combined primers are from page 323 of "Antibody Engineering" Volume 1, Edited by Roland Kontermann and Stefan Dübel). The obtained PCR products were sequenced and analyzed using the Kabat database to determine that whether the obtained sequences corresponded to the sequences of the variable regions of the murine antibody.
The relevant sequence information is as follows:
The gene sequence of the heavy chain variable region of 147# is 366bp in length, encoding 122 amino acid residues. The nucleotide sequence encoding the heavy chain variable region of 147# is as shown in SEQ ID NO: 4, and the amino acid sequence of the heavy chain variable region of 147# is shown in SEQ ID NO: 5.
The gene sequence of the light chain variable region of 147# is 321bp in length, encoding 107 amino acid residues. The nucleotide sequence encoding the light chain variable region of 147# is as shown in SEQ ID NO: 6, and the amino acid sequence of the light chain variable region of 147# is shown in SEQ ID NO: 7.
An analysis of the amino acid sequences of the light and heavy chain variable regions of the murine antibody 147# was conducted, and based on the Kabat rules, three antigencomplementarity-determining regions (CDRs) and four framework regions (FRs) of the murine antibody 147# were determined. The amino acid sequences of the heavy chain CDRs of 147# are as follows: HCDR1: KTSGYIFT (SEQ ID NO: 10), HCDR2: IGRIYP (SEQ ID NO: 11), HCDR3: ARGGEVRRDFYALDY (SEQ ID NO: 12). The amino acid sequences of the light chain CDRs of 147# are as follows: LCDR1: KASENVGTY (SEQ ID NO: 13), LCDR2: GASNRYT (SEQ ID NO: 14), LCDR3: GQRYSYPFT (SEQ ID NO: 15).
A humanized template with the best match to the FR regions of the murine antibody was selected from the Germline database. The CDR regions of the murine antibody were transplanted onto the selected humanized template to replace the original CDR regions on the humanized template. The heavy chain variable region (VH) was then recombined with the human IgG1 constant region, and the light chain variable region (VL) was recombined with the human kappa chain constant region. Additionally, based on the three-dimensional structure of the antibody, buried residues, residues that directly interact with the CDR regions, and residues that significantly affect the conformations of the VL and VH of antibodies were subjected to revertant mutations. This resulted in the heavy chain variable region of the humanized antibody (hu147VH, amino acid sequence as shown in SEQ ID NO: 21) and the light chain variable region of the humanized antibody (hu147VL, amino acid sequence as shown in SEQ ID NO: 22). hu147VH was linked to the human IgG1 constant region (amino acid sequence as shown in SEQ ID NO: 8) to form the heavy chain of the humanized antibody (hu147H, amino acid sequence as shown in SEQ ID NO: 23). hu147VL was linked to the human kappa chain constant region (amino acid sequence as shown in SEQ ID NO: 9) to form the light chain of the humanized antibody (hu147L, amino acid sequence as shown in SEQ ID NO: 24). The genes for the heavy chain and the light chain of the humanized antibody were respectively constructed into the pcDNA3.4 expression vector, transfected into Expi-293F cells for expression of protein, and the protein was purified by Protein A affinity chromatography to obtain the humanized antibody hu147. The molecular weight (whether meet the expectation) and purity (>95%) of the antibody were determined by SDS-PAGE electrophoresis and SEC-HPLC.

**Embodiment 7 Preparation of Fc-mutated Humanized Antibody (hu147mu)**

The heavy chain variable region of hu147 was linked to a mutated human IgG1 constant region (amino acid sequence as shown in SEQ ID NO: 25) to form the heavy chain of hu147mu (amino acid sequence as shown in SEQ ID NO: 26). The light chain of hu147mu was the same as that of hu147 (amino acid sequence as shown in SEQ ID NO: 24). The genes for the heavy chain and the light chain of hu147mu were constructed into the pcDNA3.4 expression vector and transfected into Expi-293F cells. The antibody hu147mu was purified by Protein G and obtained. The molecular weight of the expressed antibody was determined to be around 150 kDa by SDS-PAGE electrophoresis and SEC-HPLC, and the purity was > 95%. The antibody was then quantified, aliquoted, and stored at -80°C for future use.

**Embodiment 8 Measurement of the Binding Activity of Humanized Antibody hu147 and Mutated Humanized Antibody hu147mu to Human CD276-ECD by ELISA**

The binding affinity of the humanized antibody hu147 and the mutated humanized antibody hu147mu to human CD276-ECD was measured by ELISA. The experimental methods referred to Embodiment 2.

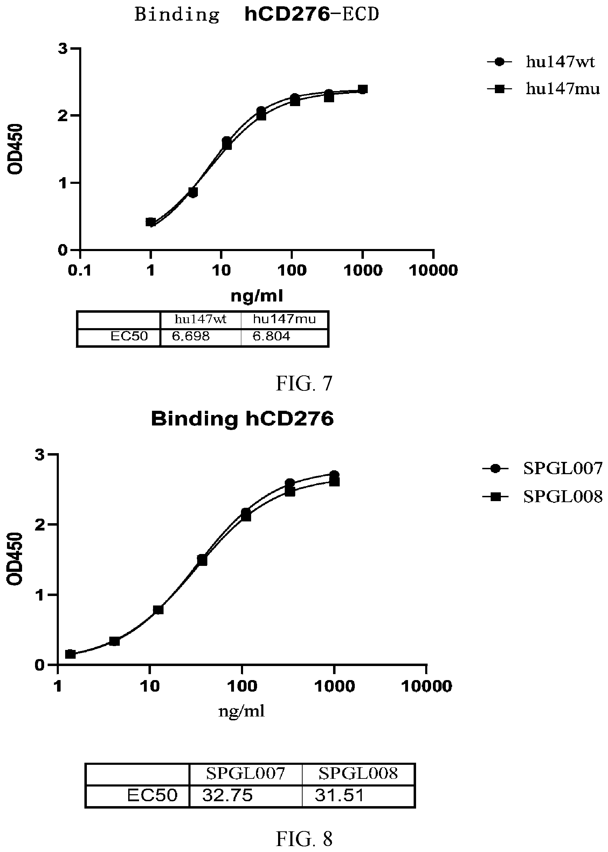

The results were shown in FIG. 7. The $EC_{50}$ values of humanized antibodies hu147 and hu147mu binding to human CD276-ECD were 6.70 ng/mL and 6.80 ng/mL, i.e., 0.04 nM and 0.05 nM, respectively, which were significantly improved compared to 147# ($EC_{50}$ = 0.26 nM). This indicates that hu147 and hu147mu have a strong binding affinity to human CD276-ECD, and that the Fc mutation does not affect the binding of the humanized antibodies to the antigen.

**Embodiment 9 Preparation of hu147-IL15 Bifunctional Molecules SPGL008 and SPGL007**

The amino acid sequence of human IL15Rsushi was as shown in SEQ ID NO: 27; the amino acid sequence of human IL15 was as shown in SEQ ID NO: 28. Human IL15Rsushi and human IL15 was linked by $(GGGGS)_6$ to form a single-chain IL15, namely EL15sc (amino acid sequence as shown in SEQ ID NO: 29). The heavy chain sequence of hu147 (amino acid sequence as shown in SEQ ID NO: 23) was linked to the human IL15sc sequence via $(GGGGS)_3$ to form the heavy chain of SPGL007 (amino acid sequence as shown in SEQ ID NO: 30). The heavy chain sequence of hu147mu (amino acid sequence as shown in SEQ ID NO: 26) was linked to the human IL15sc sequence via $(GGGGS)_3$ to form the heavy chain of SPGL008 (amino acid sequence as shown in SEQ ID NO: 31). The light chain was the same as hu147 (amino acid sequence as shown in SEQ ID NO: 24). The genes for the heavy chain and the light chain of SPGL007 were co-transfected into Expi-293F cells for expression of protein, and the protein was purified using Protein G affinity chromatography to obtain the bifunctional antibody SPGL007. The genes for the heavy chain and the light chain of SPGL008 were co-transfected into Expi-293F cells for expression of protein, and the protein was purified using Protein G affinity chromatography to obtain the bifunctional antibody SPGL008. The molecular weight of each expressed antibody was determined to be around 190 kDa by SDS-PAGE electrophoresis and SEC-HPLC, and the purity was >95%. The antibodies were then quantified, aliquoted, and stored at -80°C for future use.

**Embodiment 10 Measurement of the Binding Activity of SPGL008 and SPGL007 to Human CD276-ECD by ELISA**

The binding affinity of SPGL008 and SPGL007 to human CD276-ECD was measured by ELISA. The experimental methods referred to Embodiment 2.

The results were shown in FIG. 8. The $EC_{50}$ values of SPGL008 and SPGL007 binding to human CD276-ECD were 31.51 ng/mL and 32.75 ng/mL, i.e., 0.16 nM and 0.17 nM, respectively. Although these values are slightly lower than that of hu147 (0.05 nM) or hu147mu (0.04 nM), SPGL008 and SPGL007 still maintain relatively high binding affinity.

**Embodiment 11 SPGL008 Simultaneously Binds to Human CD276 and Human CD122/132**

An indirect enzyme-linked immunosorbent assay (ELISA) was used to measure the binding of SPGL008 and SPGL007, which can bind to CD276-ECD, to human CD122/132. The results demonstrated that SPGL008 can simultaneously bind to both CD276 and CD122/132. The method was as follows: The plates were pre-coated with human CD276-ECD protein diluted to 2 $\mu$g/mL in the coating solution (50 mM carbonate coating buffer, pH 9.6) and incubated at 4°C overnight. The plates were then blocked with 5% skim milk powder at 37°C for 2 hours. Afterward, the plates were washed 3 times with PBST, and the to-be-tested antibody, diluted in 1% BSA-PBST at various gradients, was added to the blocked plates at 100 $\mu$L per well. The plates were incubated at 37°C for 1 hour. After washing the plates 3 times with PBST, serially diluted biotinylated CD122/132 (purchased from Acro BIOSYSTEMS) was added, and the plates were incubated at 37°C for 1 hour. Following 3 additional washes with PBST, diluted HRP-conjugated streptavidin (SA) (purchased from Pierce) was added, and the plates were incubated at 37°C for 30 minutes. After 3 more washes with PBST, residual droplets were removed by blotting on absorbent paper. Then, 100 $\mu$L of TMB substrate (purchased from KPL) was added to each well, and the plates were incubated at room temperature (20 $\pm$ 5°C) in the dark for 5 minutes. Finally, 50 $\mu$L of 2 M $H_2SO_4$ was added to each well to terminate the substrate reaction, and the OD value at 450 nm was read using a microplate reader to analyze the binding ability of the to-be-tested antibody to human CD122/132.

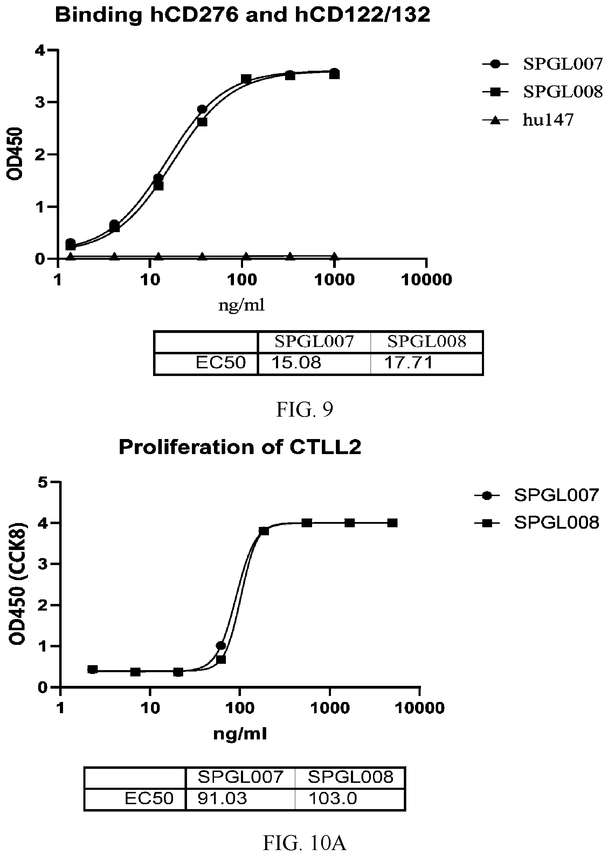

The results, shown in FIG. 9, indicated that both antibodies SPGL008 and SPGL007 exhibited excellent binding activity. Under the experimental conditions, the $EC_{50}$ values of SPGL008 and SPGL007 binding to human CD122/132 were 17.71 ng/mL and 15.08 ng/mL, i.e., 0.09 nM and 0.08 nM, respectively. As a control, hu147 could bind to CD276 (as shown in Embodiment 8), but not CD122/CD132.

**Embodiment 12 Cell Proliferation-Promoting Activity of SPGL008**

This embodiment demonstrated the biological activity of SPGL008 and SPGL007 through a CTLL2 cell proliferation assay. The method was as follows: CTLL2 cells were diluted to a concentration of $5\times10^4$/mL in 1640 medium containing 10% FBS, and 100 $\mu$L was added to each well of a cell culture plate. IL2 was diluted to 30 ng/mL in 1640 medium containing 10% FBS, followed by a 3-fold serial dilution for a total of 8 gradients, each of which was added to the culture plate containing CTLL2 cells. SPGL008 and SPGL007 were diluted to 5000 ng/mL in 1640 medium containing 10% FBS, followed by a 3-fold serial dilution for a total of 8 gradients, each of which was added to the culture plate containing CTLL2 cells. After 72 hours of incubation in a $CO_2$ cell culture incubator, relative cell numbers in each well were measured using the CCK8 assay, and the EC50 values were calculated to determine the activity of the samples.

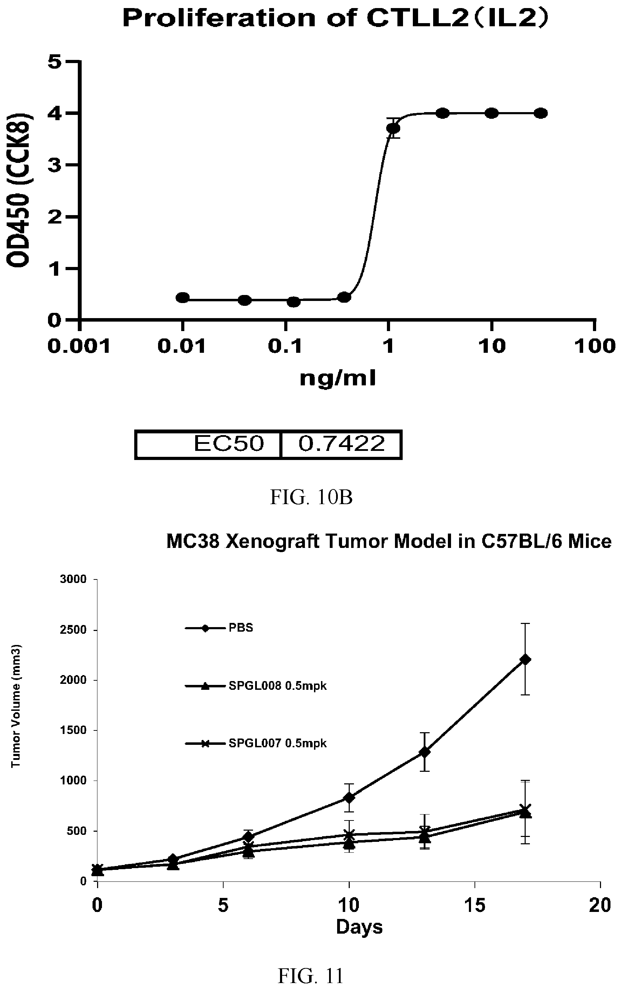

The results, shown in FIG. 10A, indicated that both SPGL008 and SPGL007 could stimulate the proliferation of CTLL2 cells, with EC50 values of 103.0 ng/mL and 91.0 ng/mL, i.e., 0.53 nM and 0.48 nM, respectively, demonstrating that SPGL008 has biological activity consistent with that of SPGL007. As a control, IL2 had an $EC_{50}$ value of 0.74 ng/mL, i.e., 0.048 nM, as shown in FIG. 10B.

**Embodiment 13 SPGL008 Exhibits Significantly Lower Toxicity in Mice Compared to SPGL007**

SPGL008 and SPGL007 were intraperitoneally injected into C57BL/6 mice (purchased from Charles River) on day 1 and day 3, with a total of two injections, each with a volume of 0.2 ml. SPGL008 and SPGL007 were administered at doses of 0.5 mg/kg, 1 mg/kg, 2 mg/kg, and 4 mg/kg. The mortality of the experimental mice was observed daily. An equal volume of PBS was administered to the control group.

The results were shown in Table 1. On day 10 of the experiment, in the SPGL008 group at doses of 0.5 mg/kg, 1.0 mg/kg, and 2.0 mg/kg, all animals survived (100% survival rate), in the SPGL008 group at a dose of 4.0 mg/kg, 70% of the animals died (30% survival rate). However, in the SPGL007 group, at doses of 4.0 mg/kg and 2.0 mg/kg, 100% of the animals died (0% survival rate), at a dose of 1.0 mg/kg, 60% of the animals died (40% survival rate), and at a dose of 0.5 mg/kg, no animals died (100% survival rate). These results indicate that SPGL008 with the Fc mutation exhibits significantly lower toxicity in experimental mice compared to SPGL007.

Table 1: Survival rates of experimental animals in different dose group

| Antibody | 0.5mg/kg | 1.0mg/kg | 2.0mg/kg | 4.0mg/kg |
|----------|----------|----------|----------|----------|
| SPGL007 | 100% | 40% | 0% | 0% |
| SPGL008 | 100% | 100% | 100% | 30% |

**Embodiment 14 SPGL008 Inhibits the Growth of Mouse Colorectal Cancer Cell MC38 Xenograft Tumors**

This embodiment evaluated the *in vivo* anti-tumor activity of SPGL008 and SPGL007 using a mouse colorectal cancer cell MC38 xenograft model. The experimental method was as follows:
Mouse colorectal cancer cells MC38 cultured *in vitro* were collected, and the concentration of the cell suspension was adjusted to $1 \times 10^7$/mL. Under sterile conditions, 100 μL of the cell suspension was inoculated subcutaneously into the right rib of C57BL/6 mice, the diameters of the xenograft tumors were measured with a vernier caliper. Once the average tumor volume reached 100-200 mm$^3$, the animals were randomly divided into groups, with 6 mice per group. Both SPGL008 and SPGL007 were administered at a dose of 0.5 mg/kg, and an equal volume of PBS was administered to the control group. All groups were administered by intraperitoneal injection twice a week, with a volume of 0.2 ml per injection, for a total of 2 weeks. During the entire experiment, the diameters of the xenograft tumors were measured three times per week, and the body weight of the mice was recorded. The tumor volume (TV) was calculated using the formula:

$$TV = 1/2 \times a \times b^2,$$

wherein a and b represented the length and width, respectively. The relative tumor volume (RTV) was calculated using the formula: RTV = Vt / V0, wherein V0 was the tumor volume measured at the time of grouping (i.e., day 0), and Vt was the tumor volume measured at each subsequent time point. The evaluation index for anti-tumor activity was the relative tumor proliferation rate, T/C (%), which was calculated as follows:

$$T/C\ (\%) = (TRTV\ /\ CRTV) \times 100.$$

The tumor growth inhibition rate (TGI) was calculated as: TGI (%) = 100 - T/C (%),
[0094]    TRTV: treatment group RTV, CRTV: negative control group RTV.
The results, as shown in FIG. 11, indicated that both SPGL008 and SPGL007 exhibited excellent anti-tumor activity, with TGIs of 76.2% and 69.4%, respectively. The difference between the two groups was not significant (p > 0.05), indicating that the Fc mutation in SPGL008 did not affect *in vivo* anti-tumor activity.

**Embodiment 15 SPGL008 Inhibits the Growth of Human Lung Cancer Cell NCI H1975 Xenograft Tumors**

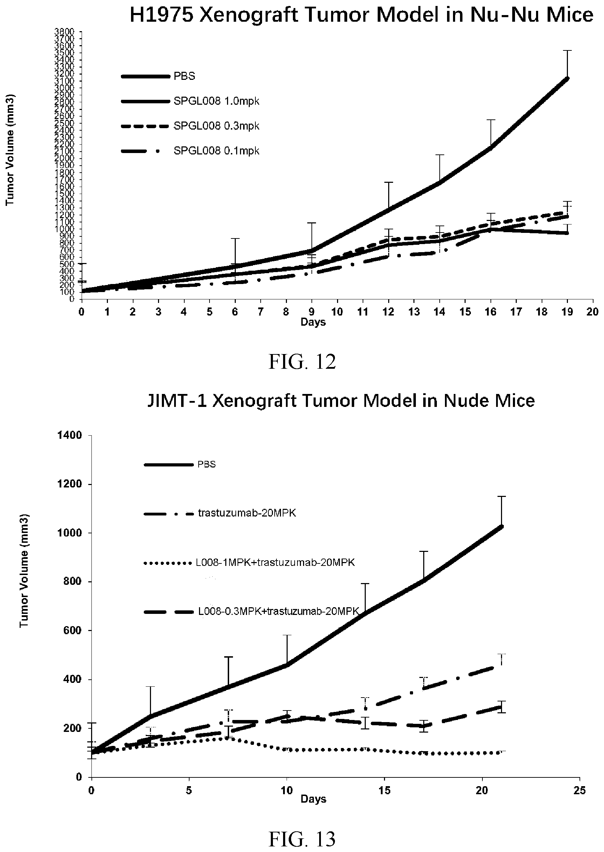

Human lung cancer cells NCI-H1975 were cultured *in vitro,* and the concentration of the cell suspension was adjusted to $8 \times 10^7$/mL. Under sterile conditions, 100μL of the cell suspension was inoculated subcutaneously into the right rib of the nude mice. After the tumor cells formed solid tumors in the mice, the diameters of the xenograft tumors were measured with a vernier caliper. Once the average tumor volume grew to 50-100 mm$^3$, the animals were randomly divided into groups. All groups were administered by intraperitoneal injection three times a week at doses of 1.0 mg/kg, 0.3 mg/kg, and 0.1 mg/kg, respectively, for a total of 6 injections. During the experiment, the diameters of the xenograft tumors were measured twice a week, and the body weight of the mice was recorded. Other experimental procedures followed that in Embodiment 14.
The results, as shown in FIG. 12, indicated that SPGL008 exhibited excellent anti-tumor activity, with TGIs of 70.0%, 60.8% and 62.5% at doses of 1.0 mg/kg, 0.3 mg/kg and 0.1 mg/kg, respectively.

**Embodiment 16 SPGL008 Combined with HER2 Monoclonal Antibody Inhibits the Growth of Human Breast Cancer Cell JIMT-1 Xenograft Tumors in Nude Mice**

Human breast cancer JIMT-1 cells cultured *in vitro* were collected, and the concentration of the cell suspension was adjusted to $8 \times 10^7$ /mL. Under sterile conditions, 100 μL of the cell suspension was inoculated subcutaneously via the right rib of the nude mice. After the tumor cells formed solid tumors in the mice, the diameters of the xenograft tumors were measured with a vernier caliper. Once the average tumor volume grew to 50-100 mm$^3$, the animals were randomly divided into groups. In monotherapy group, the HER2 monoclonal antibody (trastuzumab) was administered at a dose of 20 mg/kg. In the combination therapy group, trastuzumab was administered at a fixed dose of 20 mg/kg combined with 1.0 mg/kg or 0.3 mg/kg of SPGL008, respectively. All groups were administered by intraperitoneal injection twice a week for a total of 6 injections. During the experiment, the diameters of the xenograft tumors were measured twice a week, and the body weight of the mice was also recorded. Other experimental procedures followed that in Embodiment 15.
The results, shown in FIG. 13, indicated that the trastuzumab group exhibited a relatively weak anti-tumor effect, with a TGI of 55%, suggesting that JIMT-1 is a trastuzumab-tolerant tumor. However, SPGL008, with a dose of 1.0 mg/kg, combined with trastuzumab had a TGI reaching 90%, and 50% (3/6) of the tumors completely disappeared (CR). Even at a dose of 0.3 mg/kg, SPGL008 combined with trastuzumab had a TGI reaching 72%, demonstrating that the combination of SPGL008 and trastuzumab has a better anti-tumor effect than trastuzumab alone.

Embodiment 17 Metabolism of SPGL008 in huFcRn Transgenic Mice

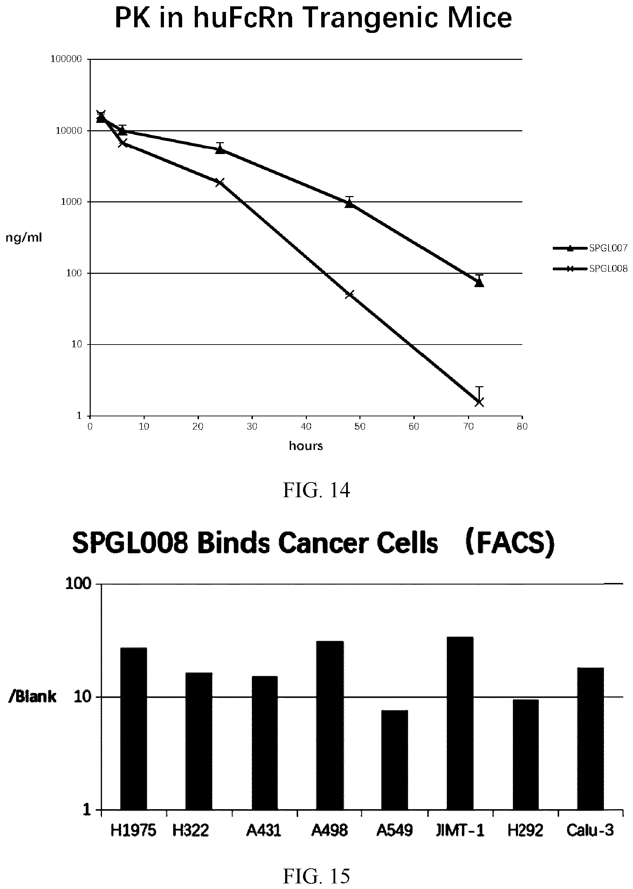

Pharmacokinetics of SPGL007 and SPGL008 were determined in human FcRn transgenic mice. The method was as follows: Eight mice were divided into two groups, and SPGL007 and SPGL008 were intraperitoneally injected at a dose of 1 mg/kg, respectively. Blood samples were collected at 2 hours, 6 hours, 24 hours, 48 hours, and 72 hours post-injection to obtain serum. After appropriate dilution, serum drug concentrations were measured using the ELISA method, which is similar to that in Embodiment 11.
The results, as shown in FIG. 14, indicated that the Fc mutation in SPGL008 significantly accelerated metabolism in human FcRn transgenic mice compared to SPGL007 containing the wild-type Fc. The half-lives of SPGL008 and SPGL007 were 10 hours and 18 hours, respectively, showing a significant difference.

**Embodiment 18 Detection of CD276 Expression in Various Tumor Cells Using SPGL008**

Flow cytometry was used for detection, and the method was nearly the same as that in Embodiment 3.
The results, as shown in FIG.15, indicated that SPGL008 could bind to various human lung cancer cells (H1975, Calu-3, A549, H322, H292), human breast cancer cells (JIMT-1), human kidney cancer cells (A498), and human skin cancer cells (A431), suggesting that SPGL008 are potential effective for the aforementioned types of tumors.

Embodiment 19 SPGL008 Exhibits Good Stability

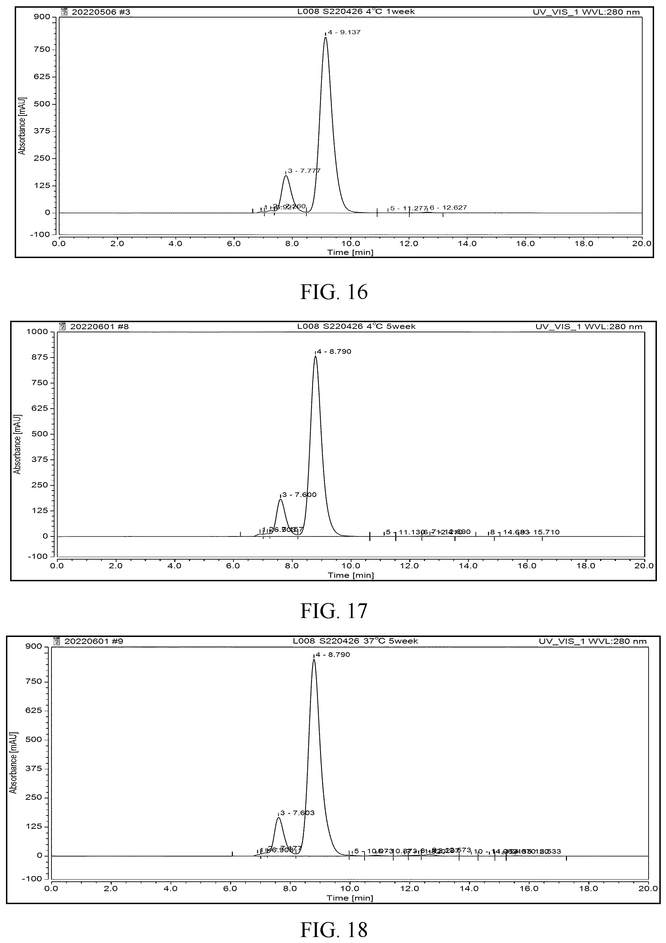

The stability of SPGL008 was assessed by measuring changes in purity after different storage times using Size Exclusion High-Performance Liquid Chromatography (SEC-HPLC). The method was as follows:
SPGL008 was analyzed using an HPLC Ultimate 3000 system (purchased from Thermo company) with a TSKgel G3000SWXL column (purchased from TSK company). The mobile phase used was PBS (pH 7.4) with a constant flow rate of 0.8 ml/min, and the sample volume was 100 $\mu$g/100 $\mu$L. The purity of the target protein was determined by calculating the percentage of the target protein in the total protein using the 280 nm absorbance peak area integration method.
The results, as shown in FIGs. 16, 17, and 18, demonstrated that SPGL008 had a purity of 84.9% at 0 week storage in PBS solution. After storage at 4°C for 5 weeks, the purity was 84.0%, with a change rate of <2%. After storage at 37°C for 5 weeks, the purity was 83.9%, with a change rate of <2%. These results indicate that SPGL008 remains stable in PBS buffer at both 4°C and 37°C.
It should be noted that the above embodiments are only used to illustrate the technical solution of the present disclosure, rather than to limit the scope of protection of the present disclosure. Although the present disclosure has been described in detail with reference to the preferred embodiments, those skilled in the art should understand that the technical solution of the present disclosure may be modified or replaced by equivalents without departing from the essence and scope of the technical solution of the present disclosure.

**Claims**

1. A monoclonal antibody, wherein the monoclonal antibody recognizes human CD276-ECD protein,
   wherein the monoclonal antibody comprises a heavy chain variable region having an amino acid sequence of SEQ ID NO: 5, and a light chain variable region having an amino acid sequence of SEQ ID NO: 7.

2. A recombinant antibody, wherein the recombinant antibody is a humanized antibody of the monoclonal antibody according to claim 1,
   wherein the recombinant antibody comprises a heavy chain variable region having an amino acid sequence of SEQ ID NO: 21, and a light chain variable region having an amino acid sequence of SEQ ID NO: 22.

3. A multifunctional recombinant antibody, wherein the multifunctional recombinant antibody comprises a heavy chain, the heavy chain of the multifunctional recombinant antibody comprises a human CD276-targeting antibody functional region, a human IgG1 constant functional domain, a human IL15 functional region, and multiple non-functional amino acid fragments for linking the functional domain and the functional regions,
   wherein the human CD276-targeting antibody functional region comprises the amino acid sequence of the heavy chain variable region of the recombinant antibody according to claim 2.

4. The multifunctional recombinant antibody according to claim 3, wherein the human IgG1 constant functional domain is a mutated human IgG1 constant region, and the mutated human IgG1 constant region has an amino acid sequence of SEQ ID NO: 25;

wherein the heavy chain of the multifunctional recombinant antibody has an amino acid sequence of SEQ ID NO: 31;
wherein the multifunctional recombinant antibody comprises a light chain having an amino acid sequence of SEQ ID NO: 24.

5. A nucleotide sequence encoding the monoclonal antibody according to claim 1, the recombinant antibody according to claim 2, or the multifunctional recombinant antibody according to claim 3 or 4, wherein the multifunctional recombinant antibody according to claim 3 or 4 is a CD276-targeting IL15 recombinant antibody.

6. An expression vector, comprising the nucleotide sequence according to claim 5.

7. A host cell, comprising the expression vector according to claim 6.

8. Use of the monoclonal antibody according to claim 1, the recombinant antibody according to claim 2, the multifunctional recombinant antibody according to claim 3 or 4; the nucleotide sequence according to claim 5, the expression vector according to claim 6, or the host cell according to claim 7 in the preparation of a biopharmaceutical composition for the treatment of tumors, wherein the multifunctional recombinant antibody according to claim 3 or 4 is a CD276-targeting IL15 recombinant antibody.

9. A biopharmaceutical composition, comprising one or more of the monoclonal antibody according to claim 1, the recombinant antibody according to claim 2, the multifunctional recombinant antibody according to claim 3 or 4, the nucleotide sequence according to claim 5, the expression vector according to claim 6, and the host cell according to claim 7, wherein the multifunctional recombinant antibody according to claim 3 or 4 is a CD276-targeting IL15 recombinant antibody.

10. A method for preparing the monoclonal antibody according to claim 1, the recombinant antibody according to claim 2, or the multifunctional recombinant antibody according to claim 3 or 4, comprising the following steps:

(1) obtaining an expression vector comprising a gene fragment of the monoclonal antibody, the recombinant antibody, or the multifunctional recombinant antibody by artificial synthesis or molecular biology methods;
(2) transfecting the expression vector into cells for expression of protein;
(3) purifying the protein to obtain the monoclonal antibody, the recombinant antibody, or the multifunctional recombinant antibody;

wherein the multifunctional recombinant antibody according to claim 3 or 4 is a CD276-targeting IL15 recombinant antibody.

Binding **hCD276**-ECD （**147#**）

| | 147# |
|---|---|
| EC50 | 38.35 |

FIG. 1

**Binding A498 Cell(147#)**

| EC50 | 59.15 |
|---|---|

FIG. 2

1, CD276-ECD
2, Domain1-Fc
3, Domain2-Fc
4, MW Marker

FIG. 3

Binding Peptides from CD276（147#）

FIG. 4

Binding Alanine substituted Peptides from CD276 (147#)

FIG. 5

Binding Different Sourced CD276-ECD

FIG. 6

Binding **hCD276**-ECD

| | hu147wt | hu147mu |
|---|---|---|
| EC50 | 6.698 | 6.804 |

FIG. 7

**Binding hCD276**

| | SPGL007 | SPGL008 |
|---|---|---|
| EC50 | 32.75 | 31.51 |

FIG. 8

## Binding hCD276 and hCD122/132

Legend:
- ● SPGL007
- ■ SPGL008
- ▲ hu147

| | SPGL007 | SPGL008 |
|---|---|---|
| EC50 | 15.08 | 17.71 |

FIG. 9

## Proliferation of CTLL2

Legend:
- ● SPGL007
- ■ SPGL008

| | SPGL007 | SPGL008 |
|---|---|---|
| EC50 | 91.03 | 103.0 |

FIG. 10A

## Proliferation of CTLL2(IL2)

| EC50 | 0.7422 |

FIG. 10B

## MC38 Xenograft Tumor Model in C57BL/6 Mice

FIG. 11

FIG. 12

FIG. 13

## PK in huFcRn Trangenic Mice

FIG. 14

## SPGL008 Binds Cancer Cells (FACS)

FIG. 15

FIG. 16

FIG. 17

FIG. 18

## INTERNATIONAL SEARCH REPORT

| | International application No. |
|---|---|
| | **PCT/CN2022/141447** |

| **A.** | **CLASSIFICATION OF SUBJECT MATTER** |
|---|---|

C07K 16/28(2006.01)i; C12N 15/13(2006.01)i; A61K 39/395(2006.01)i; A61P 35/00(2006.01)i

According to International Patent Classification (IPC) or to both national classification and IPC

| **B.** | **FIELDS SEARCHED** |
|---|---|

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNKI, NCBI, ISI Web of Science, GenBank, 中国专利生物序列检索系统, China Patents Biological Sequence Search System: 抗体, 单抗, 胞外, 域, antibody, monoclonal, extra+, domain, ecd, B7-H3, CD276, 本申请的SEQ ID NOs: 5, 7, 21和22, SEQ ID NOs: 5, 7, 21, and 22 of the present application

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | CN 114437218 A (BEIJING MENLUO BIOTECHNOLOGY CO., LTD.) 06 May 2022 (2022-05-06) <br> entire document, and in particular, claims, sequence listing, and abstract | 1-10 |

☐ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 January 2023** | **28 January 2023** |

| Name and mailing address of the ISA/CN | Authorized officer |
|---|---|
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2022/141447** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] The actual sequence listing is submitted as a file in Standard ST.26.

Form PCT/ISA/210 (continuation of first sheet) (January 2015)

## INTERNATIONAL SEARCH REPORT
### Information on patent family members

International application No.

**PCT/CN2022/141447**

| Patent document cited in search report | Publication date (day/month/year) | Patent family member(s) | Publication date (day/month/year) |
|---|---|---|---|
| CN 114437218 A | 06 May 2022 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Antibody Engineering*, vol. 1, 323